# EUROPEAN PATENT SPECIFICATION

(11) **EP 1 140 038 B1**
(45) Date of publication and mention of the grant of the patent: **19.01.2005**
(21) Application number: 99954267.3
(22) Date of filing: 17.11.1999
(51) Int. Cl.: A61K 9/70

(54) **TRANSDERMAL DRUG DELIVERY SYSTEM**
TRANSDERMALES ARZNEISTOFFVERABREICHUNGSSYSTEM
SYSTEME D'ADMINISTRATION TRANSDERMIQUE DE MEDICAMENTS

(30) Priority: 24.12.1998 GB 9828480
(43) Date of publication of application: 10.10.2001
(73) Proprietor: Dermatech Limited, London SW5 9JL (GB)
(72) Inventor: SOLOMON, Montague, Cecil, London SW3 4QT (GB); TOCILI, Biljana, London SW6 4OP (GB); SOLOMON, David, Louis, Charles, London SW5 (GB)
(74) Representative: McKelvey, Ian Edward
(86) International application number: PCT/GB1999/003811
(87) International publication number: WO 2000/038659

(56) References cited:
- EP-A- 0 276 561
- EP-A- 0 483 370
- EP-A- 0 569 338
- WO-A-92/05811
- WO-A-92/10231
- WO-A-98/09591

## Description

### Technical Field

The invention relates to transdermal drug delivery systems, that is systems for the administration of medicine through the skin of a patient and into the systemic circulation system. In this way, the medicine avoids passing through the gastro-intestinal tract and liver. Thus metabolism is to some extent avoided, and a smaller dose can be used.

### Background Art

GB 2249956 contains a useful summary of the state of the art, and discloses such systems containing super-saturated solutions of an active ingredient within an adhesive layer by use of a carefully selected mixture of solvents. EP-A-276561 discloses a method for the manufacture of a transdermal drug delivery system in which the active ingredient, piroxicam, is mixed with an alkaline agent, the mixture obtained after the alkaline agent has been added is further dissolved and dispersed in a solubilizing agent such as crotamiton, and then the mixture is blended into an aqueous adhesive base to form a homogeneous pharmaceutical composition for use in tape form. The only example given using crotamiton as the solubilizing agent has a weight ratio of piroxicam to crotamiton of 1:2, which is well in excess of saturation concentration. The mixture is blended into an aqueous adhesive base from which a transdermal tape is formed by coating the mixture onto a peel-off liner and drying.

WO-A-92/10231 discloses the use of sub-saturated solutions of the active ingredient, but only when the drug is hydrophobic. No skin penetration enhancer is contemplated.

### THE INVENTION

The invention provides for effective transdermal delivery of any number of pharmaceutically active substances at less than the saturation concentrations. The invention selects, as a solvent to create the original solution of the pharmaceutically active substance, a solvent that is both a good solvent for the drug and a good skin penetration enhancer. These solvent/enhancers are crotamiton and diethyltoluamide (DEET).

The process steps of the invention are as set out in claim 1.

By using the active substance in a ratio less than saturation level, there is a reduced risk of crystallisation, a stable system can be manufactured, and a constant rate of delivery to the patient can be obtained.

It is surprising that certain solvents act both as a skin penetration enhancer and as a solvent for the active substance. Such solvents/enhancers are crotamiton, diethyltoluamide (DEET) and mixtures thereof. The ratio of crotamiton to DEET in such a solvent mixture may be from 5:95 to 95:5% by weight of the total solvent/enhancer content depending on the delivery rate and extent of the delivery required for the active substance. By choosing a solvent/enhancer or solvent/enhancers having a boiling point higher than any drying temperature applied to the system, and controlling the drying temperature, the solvent(s) do not evaporate, the solution of the active substance never becomes saturated, and a high proportion of active substance remains in the system. The active substance/solvent(s) solution can be maintained at 20°-30° for over one month.

The pharmaceutically active substance may be:
α-Adrenergic agonists such as Adrafinil, Adrenolone, Amidephrine, Aproclonidine, Clonidine, Ephedrine, Naphasoline and Tramazoline;
β-Adrenergic agonists such as Albuterol, Clenbuterol, Clorprenaline, Methoxyphenamine and Terbuterol;
α-Adrenergic blockers such as Amosulalol, Dapiprasol, Ergoloid Mesylates, Prazosin, Terazosin, Yohimbine;
β-Adrenergic blockers such as Acebutolol, Alprenolol, Atenolol, Pindolol, Propanolol and Timolol;
Anabolics such Androstenediol, Ethylstrenol, Methandriol, Nandrolone, Oxzymesterone, Quinbolone and Stenbolone;
Analgesic (narcotic) such as Alfentanil, Benzylmorphine, Buprenorphine, Codeine, Codeine Phosphate, Dihydrocodeine, Dihydromorphine, Fentanyl, Methadone Hydrochloride, Morphine, Morphine Derivatives, Narceine, Opium, Oxycodone, Oxymorphone, Phenazocine and Sufentanil;
Analgesics (non-narcotic) such as Acetaminophen, Acetanilide, Acetylsalicylic Acid, Carbamazepine, Diflunisal, Indomethacin, Ketoprofen, Naproxen, Phenacetin, Salicylamide and Tramadol;
Androgens such as Mesterolone, 17-Methylestosterone, Testosterone and Testosterone Propionate;
Anaesthetics such as Amylocaine Hydrochloride, Bupivacaine, Lidocaine, Midazolam, Procaine, Tetracaine Hydrochloride, Thiopental Sodium and Zolamine;
Anti-acne drugs such as Algestone Acetophenide, Benzoyl Peroxide, Cyproterone, Resorcinal, Retinoic Acid and Tetroquinolone;
Anti-amebic such as Chloroquine, Chlortetracyline, Dehydroemetine, Emetine, Teclosan, Thiocarbamizine, and Tinidazole;
Antianginals such as Alprenolol, Amlodipin Diltiazem, Felodipine, Isosorbide Dinitrate, Nicardipine, Nifedipine, Nitroglycerin, Oxprenolol, Pindolol, Timoloil and Verapamil;
Antibacterial drugs such as Gentamicin, Kanamycin, Neomycin, Chloramphenicol, Chloramphenicol Pantothenate, Clindamycin, Lincomycin, Clarithromycin, Erthromycin and Cyefoserine;
Anti-estrogens such as Delmadinone Acetate, Tamoxifen and Toremifene; Antifungal drugs such as Clotrimazole, Econazole, Ketoconazole, Miconazole and Potassium Iodide;
Antihistamines such as Chlorpheniramine, Dimethindene, Pheniramine, Triprolidine and Phenothiazine;
Antihypertensive drugs such as Captopril, Enalapril, Clonidine and Minoxidil;
Anti-inflammatory drugs such as Mefenamic Acid, Flubiprofen, Ibuprofen, Indomethacin, Ketoprofen, Aspirin, Mesalamine, Olsalasine, Piroxicam and Tenoxicam;
Anti-parkinsonian drugs such as Amantadine, Levodopa, Pergolide and Pridinol;
Antipyretics such as Camphor, Menthol, Phenol, Polidocanol, Spirit of Camphor and Trimeprazine;
Anti-seborrheic drugs such as Pyrithione, Resorcinol, Selenium Sulphides and Tioxolone;
Antiseptics such as Chlorhexidine, Bismuth Iodide Oxide, Povidone Iodine, Triclosan, Triclosane Potassium, Carvacrol, p-Cresol, Chloroxine, Halquinol, Boric Acid, α-Terpineol and Chlorhexidine;
Anti-ulcerative drugs such as Cimetidine, Enprostil, Omeprasol, Ranitidine and Trimoprostil;
Anxiolytic drugs such as Buspirone, Bromazepam, Diazepam, Loxapine, and Meprobamate;
Chlorinergic agents such as Bethanechol Chloride, Physostigmine and Pyridostigmine Bromide;
Depigmentors such as Hydroquinine, Hydroquinone and Monobenzone; Estrogens such as Benzestrol, Dienestrol, Diethylstilbestrol, Dimestrol, Methestrol, Conjugated estrogenic Hormones, Equilenin, Equilin, Estradiol, EstradiolBenzoate, Estradiol 17β-Cypionate, Estriol, Estrone, Ethinyl Estradiol, Mestranol, Moxestrol, Quinestradiol and Quinestrol;
Gonadotropic hormones such as LH and PMSG;
Nootropic agents such as Aceglutamide, Antiracetam, Piracetam, Fyritinol and Tacrine.
Progestogens such as Allylestrenol, Anagestone, Chlormadinone Acetate, Delmadinone Acetate, Demegestone, Desogestrel, Dimethisterone, Dydogesterone, Ethisterone, Ethynodiol, Flurogestone Acetate, Gestodene, Gestodene Caprolate, Haloprogesterone, 17-Hydroxy-16-methylene-progesterone, 17α-Hydroxyprogesterone, 17-α-Hydroxygesterone Caprolate, Lynestrenol, Medrogestone, Medroxyprogesterone, Megestrol Acetate, Melengestrol, Norethisterone, Norethisterone Acetate, Noretynodrel, Norgesterone, Norgestimate, Norgestrel, Norgestrienone, Norvinistyerone, Pentagestrone, Progesterone, Promegestrone, Quingestrone and Trengestone;
Respiratory stimulants such as Almitrine, Doxapram, Lobeline, Sodium Succinate and Tacrine;
Vitamins, vitamin sources and vitamin extracts such as Vitamins A, B, C, D, E and K and derivatives thereof, Calciferols, Glycyrrhiza and Mecobalamin; or
Vulnerary agents such as Acetylcysteine, Allantoin, Asiaticoside, Cadexomer Iodine, Chitin, Dextranomer and Oxaceprol.

The solvent/enhancer is Crotamiton, Diethyltoluamide (DEET), or a mixture thereof. If desired the solution obtained in step (a) can also incorporate at least one of: Transcutol (Diethylene glycol monoethyl ether), Labrafil M1944CS (unsaturated polyglycolysed glycerides), Labrasol (Glyceryl and polyethylene glycol esters), Tea-tree oil (Oil of Melaleuca), Propylene Glycol, MP DIOL (2-Methyl-1,3-Propanediol) and Polyethylene Glycol.

It will be appreciated that the amount of active substance to be incorporated in the delivery system is dependent or governed by the drug composition and/or concentration, the desired therapeutic effect for a patient, and the period for which the delivery system is to provide a therapeutic drug level. Preferably, the active substance is present in an amount from 0.1% to 50% by weight of the coating material (i.e. an aqueous emulsion or adhesive solution). More preferably, 0.3% to 30% by weight of the coating material.

The active substance is generally incorporated in the solvent/enhancer at room temperature (25°C or below) and in a ratio less than 90% of saturation level to prevent crystal formation during storage. Dissolution may be aided by sonication or warming. The resulting solution can be added slowly to the adhesive which may be in the form of an aqueous dispersion or a solution, and mixed. The adhesive can be an acrylate, silicone or polyisobutylene. An adhesive thickener may be added to the mixture at about a 50% solution/water mix to produce a thicker spreading solution for reverse roll coating or knife over roll coating.

The resulting delivery system may then be coated onto a release liner, which may be a siliconised polyester such as type FL 2000 (commercially available), or paper, which naturally is impermeable to the active substance. The system can then be dried by circulating hot air, and laminated onto a backing sheet, which may be a 3M Health Care Type 1220, the backing sheet naturally being impermeable to the active substance. The layer may be coated to a wet-coat thickness of from 20 to 500µm. Alternatively, the delivery system mixture may be spread or coated onto the backing sheet, and then laminated to the release liner. The hot air circulation may be effected at gradually increased temperatures from 50°C to 140°C.

### DRAWING

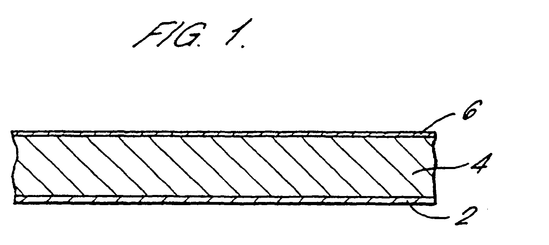

Fig. 1 is section through an adhesive tape for application to the skin of a patient comprising a drug delivery system according to the invention. A delivery system comprising an active substance, adhesive arid solvent/skin penetration enhancer 4 is coated as a layer onto a siliconized release paper 2 and laminated onto a backing strip 6.

The following Examples of ingredients in parts by weight may be used in the production of delivery systems as described above (Eg 1 does not fall within the scope of the invention):

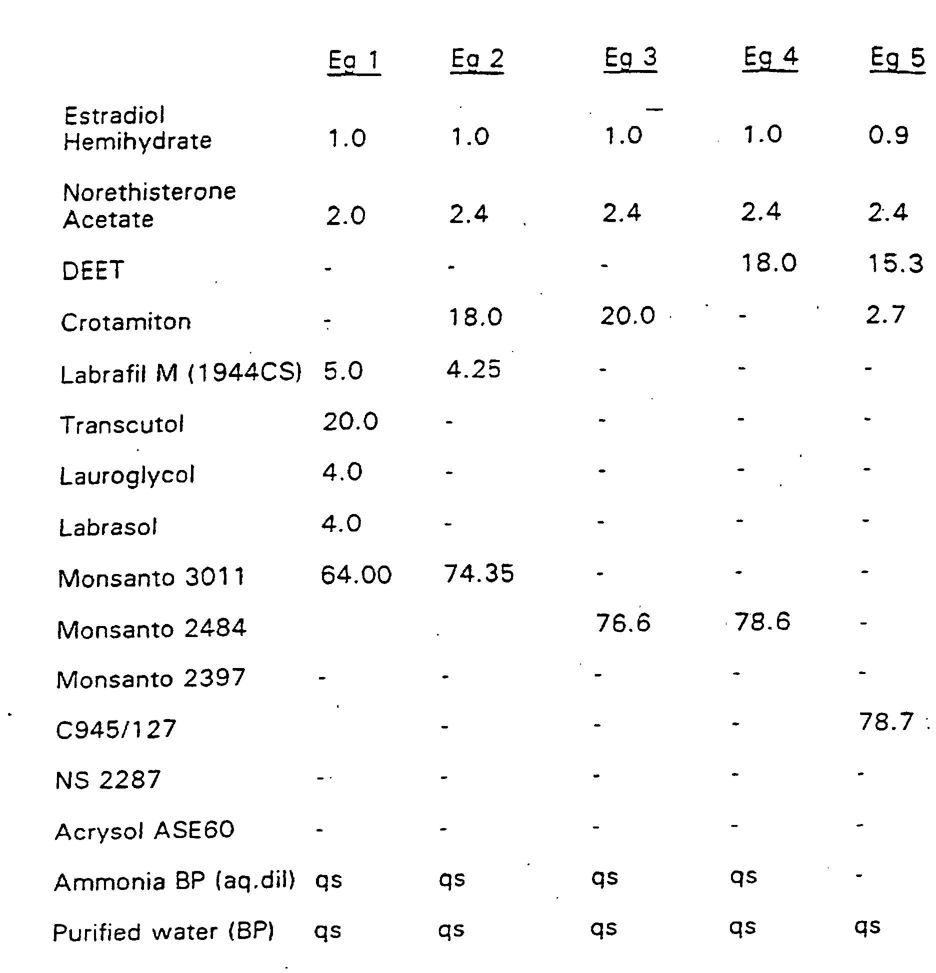

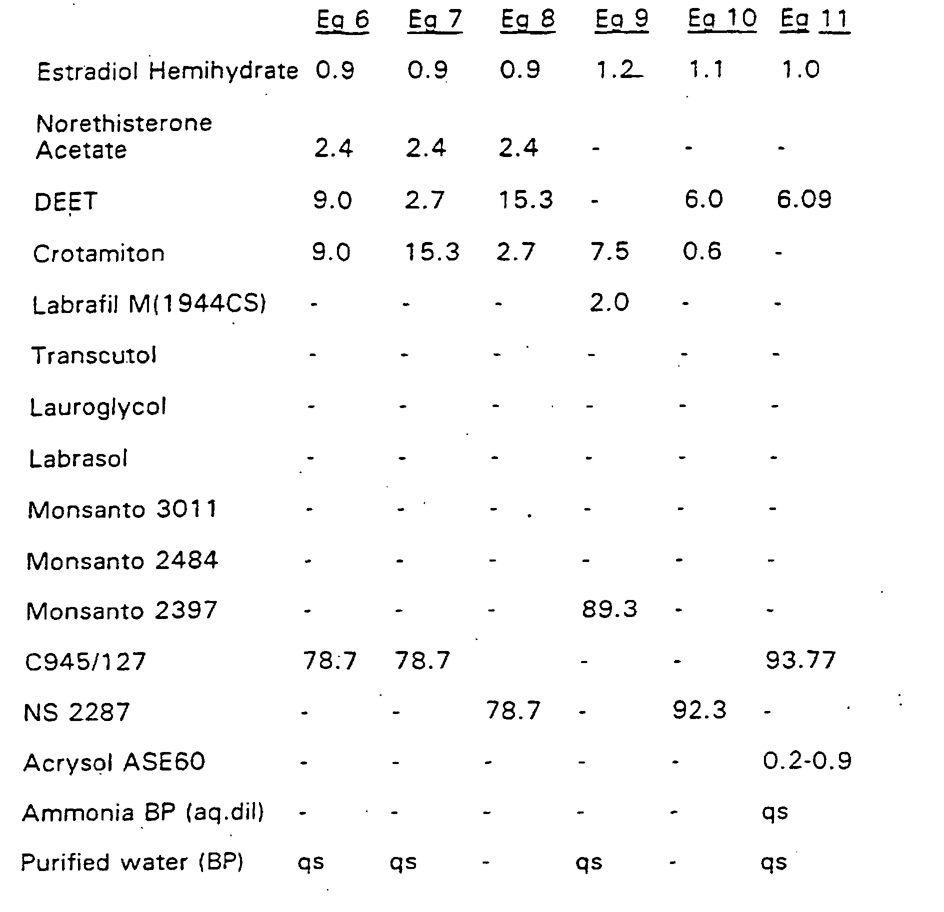

### Manufacturing Method (illustrative)

### A) Delivery System using adhesive - aqueous emulsion

The active substance is dissolved in the solvent/enhancer by means of heating and mixing over a 45 °-55 °C water bath with agitation. When the solution is optically clear, it is checked microscopically for absence of crystals.

The adhesive is weighed into a separate mixing vessel, diluted with water if necessary over a period not exceeding 30 mins to achieve the requisite viscosity. The active substance/solvent solution is gradually added to the adhesive solution with mixing. The pH is adjusted to 6.5-7.5 and a thickener is added (if appropriate) to obtain a suitable viscosity (eg 900-1000 cps) for the selected coating process such as reverse roll coating or knife over roll coating.

The resultant aqueous emulsion is coated onto a release liner (typical coating thickness 20-500 µm), and dried by passing in sequence through ovens at 50-140°C. The product is then laminated onto a backing sheet.

### B) Delivery system using an adhesive solution

The active substance is dissolved in a solvent/enhancer by means of heating and mixing as described above. The adhesive is weighed in a separate vessel and the active substance/solvent solution is gradually added to the solution of adhesive with mixing. The resultant adhesive solution is coated onto a release liner, dried by passing in sequence through ovens at 50-140°C. The product is then laminated onto a backing sheet.

### In-vitro drug delivery through the skin

In-vitro skin permeation experiments with human skin have been on systems made from the above ingredients carried out using Franz-type diffusion cells, and the studies were carried out on a Hanson Microette system.

Dermatomed human skin sections were mounted onto the Franz cells and transdermal drug delivery systems mounted on tape backings (1.5cm²) were placed on the stratum corneal surface of the skin. Each Franz cell contained 7ml of ethanol phosphate buffered saline as the receptor medium, maintained at 32°C. At predetermined time intervals 0.7ml of the receptor solution was sampled and an equal amount replaced.

The samples were analysed by High Pressure Liquid Chromatography.

The skin mass transport of Estradiol and Norethisterone Acetate has been found to be enhanced by the solvent/skin penetration enhancer DEET and/or Crotamiton in a concentration below saturation. Further, the active substance flux profile follows the solvent flux profile, the latter showing high skin penetration flux during the first 20 hours of application.

### Indications

The main indications are in both peri-menopausal and menopausal women for the control in the former of the symptoms of the menopause such as hot flushes, sweating and the other symptoms of the peri-menopause, and in the case of the menopause the prevention of osteoporosis and cardiac events such as coronary thrombosis.

## Claims

1. A method for the manufacture of a transdermal drug delivery system, which comprises the successive steps of :
(a) dissolving a pharmaceutically active substance in a solvent which is one or more of the skin penetration enhancers crotamiton and diethyltoluamide (DEET) to form a solution of active substance in the skin penetration enhancer(s) at a concentration less than saturation;
(b) mixing the resulting solution with an adhesive in the form of a solution or an aqueous dispersion;
(c) forming the mixture obtained in (b) into a coating on a release liner or backing sheet; and
(d) drying the coating at a temperature less than the boiling point of the skin penetration enhancer or enhancers used as solvent in the dissolution step (a).

2. A method according to claim 1, wherein the solvent used in step (a) comprises the skin penetration enhancers crotamiton and DEET in a ratio of from 5:95% to 95:5% by weight.

3. A method according to claim 1 or claim 2, wherein the solution obtained in step (a) additionally incorporates at least one of: transcutol (diethylene glycol monoethyl ether), Labrafil M1944CS (unsaturated polyglycolysed glycerides), Labrasol (glyceryl and polyethylene glycol esters), lauroglycol (propylene glycol laurate), tee tree oil (oil of Melaleuca), propylene glycol, MP DIOL (2-methyl-1,3-propanediol) and polyethylene glycol.

4. A method according to any preceding claim, wherein the active substance is dissolved in step (a) in the skin penetration enhancer(s) to form a solution at a concentration of less than 90% of saturation.

5. A method according to any preceding claim, wherein the active substance is estradiol or a mixture of estradiol and norethisterone acetate.

6. A method according to any preceding claim, wherein the adhesive is a solution or aqueous dispersion of an acrylate or polyisobutylene or silicone adhesive.

7. A method according to any preceding claim, wherein the coating is formed in step (c) on a release liner and the coating, after drying in step (d), is laminated on to a backing sheet.

8. A method according to any of claims 1 to 6, wherein the coating is formed in step (c) on a backing sheet and the coating, after drying in step (d), is laminated on to a release liner.

9. A method according to any preceding claim, wherein the drying temperature of step (d) is increased gradually from 50°C to 140°C.

## Patentansprüche

1. Verfahren für die Herstellung eines Systems für eine transdermale Arzneimittelabgabe, das die folgenden Schritte aufweist:
(a) Auflösen einer pharmazeutisch aktiven Substanz in einem Lösungsmittel, das eines oder mehrere der Hautdurchdringungsverstärker Crotamiton und Diethyltoluamid (DEET) ist, um eine Lösung einer aktiven Substanz in dem (den) Hautdurchdringungsverstärker(n) bei einer Konzentration geringer als der Sättigung zu bilden;
(b) Mischen der sich ergebenden Lösung mit einem Klebstoff in der Form einer Lösung oder einer wässrigen Dispersion;
(c) Bilden der in (b) erhaltenen Mischung zu einer Beschichtung auf einem Abgabestreifen oder einer Trägerschicht; und
(d) Trocknen der Beschichtung bei einer Temperatur niedriger als dem Siedepunkt des/der als Lösungsmittel in dem Lösungsschritt (a) verwendeten Hautdurchdringungsverstärkers oder -verstärker.

2. Verfahren nach Anspruch 1, wobei das in Schritt (a) verwendete Lösungsmittel die Hautdurchdringungsverstärker Crotamiton und DEET in einem Verhältnis von 5:95 Gew.-% bis 95:5 Gew.-% enthält.

3. Verfahren nach Anspruch 1 oder Anspruch 2, wobei die in Schritt (a) erhaltene Lösung zusätzlich mindestens eines der Folgenden enthält: Transcutol (Diethylen-Glycol-Monoethyl-Ether), Labrafil M1944CS (ungesättigte polyglycolisierte Glyceride), Labrasol (Glyceryl- und Polyethylen-Glycol-Ester), Lauroglycol (Propylen-Glycol-Laurat), Teebaumöl (Öl von Melaleuca), Propylen-Glycol, MP DIOL (2-Methyl-1,3-Propandiol) und Polyethylen-Glycol.

4. Verfahren nach einem der vorhergehenden Ansprüche, wobei die aktive Substanz in Schritt (a) in dem (den) Hautdurchdringungsverstärker(n) gelöst ist, um eine Lösung bei einer Konzentration von weniger als 90% der Sättigung zu bilden.

5. Verfahren nach einem der vorhergehenden Ansprüche, wobei die aktive Substanz Estradiol oder eine Mischung aus Estradiol und Norethisteron-Acetat ist.

6. Verfahren nach einem der vorhergehenden Ansprüche, wobei der Klebstoff eine Lösung oder eine wässrige Dispersion aus einem Acrylat oder Polyisobutylen oder Silikon-Klebstoff ist.

7. Verfahren nach einem der vorhergehenden Ansprüche, wobei die Beschichtung in Schritt (c) auf einem Abgabestreifen gebildet wird und die Beschichtung, nach dem Trocknen in Schritt (d), auf eine Trägerschicht laminiert wird.

8. Verfahren nach einem der Ansprüche 1 bis 6, wobei die Beschichtung in Schritt (c) auf einer Trägerschicht gebildet wird und die Beschichtung, nach dem Trocknen in Schritt (d), auf einen Abgabestreifen laminiert wird.

9. Verfahren nach einem der vorhergehenden Ansprüche, wobei die Trocknungstemperatur des Schritts (d) schrittweise von 50°C auf 140°C erhöht wird.

## Revendications

1. Procédé pour la fabrication d'un système de délivrance transdermique de substance médicamenteuse, qui comprend les étapes successives consistant :
(a) à dissoudre une substance pharmaceutiquement active dans un solvant qui est un ou plusieurs des promoteurs d'absorption cutanée crotamiton et diéthyltoluamide (DEET) pour former une solution de substance active dans le(s) promoteur(s) d'absorption cutanée à une concentration inférieure à la saturation ;
(b) à mélanger la solution obtenue avec un adhésif sous la forme d'une solution ou d'une dispersion aqueuse ;
(c) à façonner le mélange obtenu en (b) en un revêtement sur une bande à détacher ou une feuille renfort ; et
(d) à sécher le revêtement à une température inférieure au point d'ébullition du ou des promoteur(s) d'absorption cutanée utilisé(s) en tant que solvant dans l'étape de dissolution (a).

2. Procédé selon la revendication 1, dans lequel le solvant utilisé dans l'étape (a) comprend les promoteurs d'absorption cutanée crotamiton et DEET en un rapport de 5:95 % à 95:5 % en poids.

3. Procédé selon la revendication 1 ou la revendication 2, dans lequel la solution obtenue dans l'étape (a) incorpore de plus au moins l'un parmi : le transcutol (éther monoéthylique de diéthylèneglycol), le Labrafil M1944CS (glycérides polyglycosylés insaturés), le Labrasol (esters de glycéryle et de polyéthylèneglycol), le lauroglycol (laurate de propylèneglycol), l'huile essentielle d'arbre à thé (huile de Melaleuca), le propylèneglycol, le MP DIOL (2-méthyl-1,3-propanediol) et un polyéthylèneglycol.

4. Procédé selon l'une quelconque des revendications précédentes, dans lequel la substance active est dissoute dans l'étape (a) dans le(s) promoteur(s) d'absorption cutanée pour former une solution à une concentration inférieure à 90 % de saturation.

5. Procédé selon l'une quelconque des revendications précédentes, dans lequel la substance active est l'estradiol ou un mélange d'estradiol et d'acétate de noréthistérone.

6. Procédé selon l'une quelconque des revendications précédentes, dans lequel l'adhésif est une solution ou une dispersion aqueuse d'un adhésif acrylate ou polyisobutylène ou silicone.

7. Procédé selon l'une quelconque des revendications précédentes, dans lequel le revêtement est formé dans l'étape (c) sur une bande à détacher, et le revêtement, après séchage dans l'étape (d), est stratifié sur une feuille renfort.

8. Procédé selon l'une quelconque des revendications 1 à 6, dans lequel le revêtement est formé dans l'étape (c) sur une feuille renfort, et le revêtement, après séchage dans l'étape (d), est stratifié sur une bande à détacher.

9. Procédé selon l'une quelconque des revendications précédentes, dans lequel la température de séchage de l'étape (d) est augmentée progressivement de 50 °C à 140 °C.
